# EUROPEAN PATENT APPLICATION

(11) **EP 4 241 771 A2**
(43) Date of publication of application: **13.09.2023**
(21) Application number: 23166113.3
(22) Date of filing: 10.11.2014
(51) Int. Cl.: A61K 31/4245, A61K 45/06, A61K 9/00, A61K 9/107, A61K 9/14, A61K 9/16, A61K 47/40, A61K 47/14, A61P 31/14

(54) **METHODS AND COMPOSITIONS FOR TREATING SEPSIS**

(30) Priority: 08.11.2013 US 201361901656 P; 08.10.2014 US 201462061391 P
(62) Divisional of application: 14860922.5
(71) Applicant: Antivirus Therapeutics, Princeton Junction, NJ 08550 (US)
(72) Inventor: GUMASTE, Anand, New Jersey, 08550 (US); BYRON, David A., New Jersey, 08648 (US)
(74) Representative: FRKelly

(57) **Abstract**

In an aspect, the invention relates to compositions and methods for treating sepsis, including but not limited neonatal sepsis and sepsis related to infection. In an aspect, the invention relates to compositions and methods for attenuating adverse conditions of sepsis resulting from enterovirus infection. In an aspect, the invention relates to improved formulations of antiviral agents such as pleconaril for the treatment, alleviation and prevention of conditions associated with sepsis.

## Description

### CROSS REFERENCE TO RELATED PATENT APPLICATIONS

This application claim priority to U.S. Provisional Patent Application No. 61/901,656 filed November 8, 2013 which is herein incorporated by reference in its entirety, and also claims priority to U.S. Provisional Application No. 62/061,391 filed October 8, 2014 which is herein incorporated by reference in its entirety.

### BACKGROUND

Sepsis is a potentially life-threatening complication of an infection. Sepsis occurs when the body has an overwhelming immune response to infection triggering inflammatory responses throughout the body. This inflammation can trigger a cascade of changes that can damage multiple organ systems, causing them to fail. If sepsis progresses to septic shock, blood pressure drops dramatically, which may lead to death. Anyone can develop sepsis, but it's most common and most dangerous in older adults, young children or those with weakened immune systems. Early treatment of sepsis, usually with antibiotics and large amounts of intravenous fluids, may improve chances for survival.

Many doctors view sepsis as a three-stage syndrome, starting with sepsis and progressing through severe sepsis to septic shock. The goal is to treat sepsis during its mild stage, before it becomes more dangerous. To be diagnosed with sepsis, a patient must exhibit at least two of the following symptoms: modified body temperature, elevatedheart rate, elevated respiratory rate, or probable or confirmed infection. A diagnosis of severe sepsis is made if the patient exhibits at least one of the following signs and symptoms which indicate an organ may be failing: significantly decreased urine output, abrupt change in mental status, decrease in platelet count, difficulty breathing, abnormal heart pumping function, abdominal pain. In order to be diagnosed with with septic shock, a patient must have the signs and symptoms of severe sepsis plus extremely low blood pressure that doesn't adequately respond to simple fluid replacement.

While any type of infection bacterial, viral or fungal can lead to sepsis, the most likely varieties include pneumonia, abdominal infection, kidney infection, bloodstream infection (bacteremia).

Neonatal sepsis is sepsis in babies less than 4 weeks old, typically caused by a bacteria, but sometimes by virus. Since neonates have immature immune systems they are particularly susceptible to infection. A serious consequence of neonatal sepsis is neonatal meningitis.

Sepsis is also a major cause of death during the first few months of life, and approximatley 13-15% of all neonatal deaths is a result of neonatal sepsis. The mortality rate of neonatal sepsis can be as high as 50% for infants who are not treated or when treatment is not begun quickly. As soon as sepsis is suspected neonates are started on a course of antibiotics until culture results are returned. Unfortunately antibiotics do not work on sepsis caused by viral infection.

Enteroviruses (EV) (family Picornaviridae, genus *Enterovirus*) are among the most common viruses infecting humans. Newborn infants, because of the immaturity of their immune response mechanisms, are considered to be at a higher risk of infection and serious clinical manifestations due to EV infections. EV infections in neonates are common, especially during the summer and fall months, and lead to a wide range of manifestations, from mild febrile illness to severe, potentially fatal sepsis like conditions with multi-organ failure, referred to as "neonatal enteroviral sepsis." As with bacterial meningitis, enteroviral meningitis is a serious manifestation of the disease. Currently patients are provided with supportive care but there is no specific antiviral treatment for sepsis caused by a viral infection.

Serious consequences can result from systemic enteroviral infection including meningoencephalitis, cardiovascular collapse, myocarditis or hepatitis. Myocarditis, or hepatitis carry high mortality rates. Mortality among neonates with myocarditis alone is reported to be 30-50%. When overwhelming hepatic failure results, excess of 80% of such patients will die within one to three weeks of onset of symptoms despite aggressive medical management.

Sepsis is more common and more dangerous for individuals that are are very young or very old, have a compromised immune system, are already very sick, often in a hospital's intensive care unit, have wounds or injuries, such as burns, and/or have invasive devices, such as intravenous catheters or breathing tubes

Sepsis ranges from less to more severe. As sepsis worsens, blood flow to vital organs, such as the brain, heart and kidneys, becomes impaired. Sepsis can also cause blood clots to form in organs and in the arms, legs, fingers and toes, leading to varying degrees of organ failure and tissue death (gangrene).

Most people recover from mild sepsis, but the mortality rate for septic shock is nearly 50 percent. Also, an episode of severe sepsis may place individuals at higher risk of future infections.

Diagnosing sepsis can be difficult because its signs and symptoms can be caused by other disorders. Doctors often order a battery of tests to try to pinpoint the underlying infection. Typically a blood test is conducted to test for a variety of factors: evidence of infection, clotting problems, abnormal liver or kidney function, impaired oxygen availability, and/or electrolyte imbalances.

Other laboratory tests may include urine tests (for signs of bacteria), sample of wound secretions (if a wound appears to be infected testing a sample of the wound's secretions can help show what type of antibiotic might work best), or respiratory secretions (if a patient is coughing up mucus (sputum), it may be tested to determine what type of germ is causing the infection).

In certain situations where the site of infection is not obvious, the doctor may order one or more of the following imaging test: X-ray. (X-rays are good for visualizing problems in the lungs), computerized tomography (CT) (infections in the appendix, pancreas or bowels are easier to see on CT scans. This technology takes X-rays from a variety of angles and combines them to depict cross-sectional slices of the body's internal structures, ultrasound (i.e. particularly useful to check for infections in internal organs such as gallbladder or ovaries); magnetic resonance imaging (MRI) (MRIs may be helpful in identifying soft tissue infections, such as abscesses within the spine).

Early and aggressive treatment boosts chances of surviving sepsis. People with severe sepsis require close monitoring and treatment in a hospital intensive care unit. If patients have severe sepsis or septic shock, lifesaving measures may be needed to stabilize breathing and heart function.

Medications available for treatment include antibiotics, and vasopressors. Treatment with antibiotics begins immediately usually even before the infectious agent is identified. Initially the patient receives broad-spectrum antibiotics, which are effective against a variety of bacteria. And usually, the antibiotics are administered intravenously (IV). If blood pressure remains too low even after receiving intravenous fluids, a patient may be given a vasopressor medication, which constricts blood vessels and helps to increase blood pressure. Other medications that may be administered include low doses of corticosteroids, insulin to help maintain stable blood sugar levels, drugs that modify the immune system responses, and painkillers or sedatives.

As previously mentioned, enteroviruses are frequently involved in causing sepsis. Enteroviruses include coxsackieviruses A1 to A21, A24, and B1 to 6, echoviruses (enteric cytopathic human orphan viruses) 1 to 7, 9, 11 to 21, 24 to 27, and 29 to 33, enteroviruses 68 to 71, 73 to 91, and 100 to 101 and polioviruses types 1 to 3.

Enteroviruses, along with rhinoviruses and human parechoviruses, are picornaviruses (pico, or small, RNA viruses). Human parechoviruses types 1 and 2 were previously named echovirus 22 and 23 but have now been reclassified. All enteroviruses are antigenically heterogeneous and have wide geographic distribution.

Enteroviruses are shed in respiratory secretions and stool and sometimes are present in the blood and CSF of infected patients. Infection is usually transmitted by direct contact with respiratory secretions or stool but can be transmitted by contaminated environmental sources (eg, water). Enteroviral diseases or epidemics in the US occur in summer and fall. Infection transmitted by a mother during delivery can cause severe disseminated neonatal infection, which may include hepatitis or hepatic necrosis, meningoencephalitis, myocarditis, or a combination.

Intact humoral immunity and B-cell function are required for control of enteroviral disease. Severe enteroviral infections (often manifesting as a slowly progressive meningoencephalitis) occur in patients with agammaglobulinemia but usually not in those with other immune deficiencies.

Enteroviruses cause various syndromes: epidemic pleurodynia, hand-foot-and-mouth disease, herpangina, and poliomyelitis are caused almost exclusively by enteroviruses. Other disorders (eg, aseptic meningitis, myopericarditis) may be caused by enteroviruses or other organisms.

Aseptic meningitis is most common among infants and children. In infants and young children, the cause is frequently a group A or B coxsackievirus, an echovirus, or a human parechovirus. In older children and adults, other enteroviruses as well as other viruses may cause aseptic meningitis.

Myopericarditis is a cardiac infection that may occur at any age, but most patients are 20 to 39 years old. Patients may present with chest pain, arrhythmias, heart failure, or sudden death. Recovery is usually complete, but some patients develop dilated cardiomyopathy. Diagnosis may require reverse transcriptase (RT)-PCR of myocardial tissue. Myocarditis neonatorum (cardiac infection at birth) is caused by group B coxsackieviruses, some echoviruses, and human parechoviruses. It causes fever and heart failure and has a high mortality rate.

Enteroviruses are often implicated in causing rashes. Certain coxsackieviruses, echoviruses, and human parechoviruses may cause rashes, often during epidemics. Rashes are usually nonpruritic, do not desquamate, and occur on the face, neck, chest, and extremities. They are sometimes maculopapular or morbilliform but occasionally hemorrhagic, petechial, or vesicular. Fever is common. Aseptic meningitis may develop simultaneously.

Respiratory infections resulting from enteroviruses exhibit symptoms including fever, coryza, pharyngitis, and, in some infants and children, vomiting and diarrhea. Bronchitis and interstitial pneumonia occasionally occur in adults and children.

Additional infections with which enteroviruses are associated include those related to bone marrow transplantation, chronic enterovirus meningoencephalitis and agammaglobulinemia or hypogammaglobulinemia (CEMA) and rhinovirus pneumonia.

Despite advances in understanding the physiology and pathophysiology of sepsis, including neonatal sepsis, there is still a scarcity of compounds that are both potent, efficacious, and safe in the treatment of sepsis. These needs and other needs are satisfied by the present invention.

### SUMMARY

Disclosed herein are methods and compositions for the treatment of sepsis in a subject in need thereof, the method comprising, administering to the subject a composition comprising pleconaril. In an aspect, the sepsis is caused by infection, by enterovirus, and/or in an aspect, the sepsis is neonatal sepsis.

Disclosed herein are methods and compositions for the treatment of sepsis in a subject in need thereof, the method comprising, administering to the subject a composition comprising pleconaril wherein the pleconaril is administered via oral administration, transdermal administration, administration by inhalation, nasal administration, topical administration and other routes of administration known to those skilled in the art.

Disclosed herein are methods and compositions for the treatment of sepsis in a subject in need thereof, wherein the compositions comprising pleconaril comprise a parenteral formulation of pleconaril including pharmaceutically acceptable oils, pharmaceutically acceptable ester mixtures, saturated fatty acids, hydrofluorocarbons, and/or combinations thereof.

Disclosed herein are methods and compositions for the treatment of sepsis in a subject in need thereof, wherein the compositions comprising pleconaril comprise nanoemulsions of pleconaril.

Disclosed herein are methods and compositions for the treatment of sepsis in a subject in need thereof, wherein the compositions comprising pleconaril are incorporated into polymeric phospholipid micelles.

Disclosed herein are methods and compositions for the treatment of sepsis in a subject in need thereof, wherein the compositions comprising pleconaril comprise an oral formulation wherein the oral formulation comprises colloidal particles of pleconaril, optionally combined with carrier particles.

Disclosed herein are methods and compositions for the treatment of sepsis in a subject in need thereof, wherein the compositions comprising pleconaril comprise an oral formulation wherein the oral formulation comprises particles of pleconaril, incorporated into cyclic oligosaccharides.

Disclosed herein are methods and compositions for the treatment of sepsis in a subject in need thereof, wherein the compositions comprising pleconaril comprise an oral formulation wherein the oral formulation comprises an oil-in-water microemulsion.

Disclosed herein are methods and compositions for the treatment of sepsis in a subject in need thereof, wherein the compositions comprising pleconaril comprise an oral formulation wherein the oral formulation comprises spray-dried emulsions of pleconaril combined with digestible oil.

Disclosed herein are methods and compositions for the treatment of sepsis in a subject in need thereof, the methods comprising, administering to the subject a composition comprising pleconaril wherein the pleconaril is incorporated into an extended release composition.

Disclosed herein are methods and compositions for the treatment of sepsis in a subject in need thereof, the methods comprising, administering to the subject a composition comprising pleconaril wherein the compositions further comprise a pharmaceutically acceptable carrier.

Disclosed herein are methods and compositions for the treatment of sepsis in a subject in need thereof, the methods comprising, administering to the subject a composition comprising pleconaril wherein the subject is considered at-risk. In an aspect, the at-risk subject comprises an infant, an individual aged 65 or older, an individual in an immunocomprised state, or an individual undergoing a transplant procedure.

### BRIEF DESCRIPTION OF THE FIGURES

The accompanying figures, which are incorporated in and constitute a part of this specification, illustrate several aspects and together with the description serve to explain the principles of the invention.
Figure 1 presents a Table showing the maternal and neonatal characteristics of enrolled subjects in a study (Example 1) to investigate efficacy of pleconaril as antiviral therapy for neonatal enterovirus infections.
Figure 2 presents viral culture results among enterovirus-confirmed subjects.
Figure 3 shows the time to culture negativity for all anatomic sites (oropharynx, rectum, urine, and serum) combined among the 19 subjects in the treatment group and 7 in the placebo group who were culture-positive from any site. A subject was considered culture-negative on a given study day only when cultures assays from all sampled sites on that day were negative.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION

Sepsis is a potentially life-threatening complication of an infection, it occurs as a result of the body's overwhelming immune response to infection. Resulting inflammation can trigger a cascade of changes that can damage multiple organ systems, causing them to fail. Left untreated, or allowed to progress, sepsis may cause a patient to suffer septic shock which may result in death. Although sepsis can occur in anyone, it is most common and most dangerous in older adults or those with weakened immune systems. Sepsis is also a concern for newborns: neonatal sepsisa blood infection that occurs typically in an infant less than 90 days old.

The incidence of sepsis appears to be increasing in the United States. The causes of this increase may include: an aging population (Americans are living longer, which is swelling the ranks of the highest risk age group, people older than 65), drug-resistant bacteria (many types of bacteria can resist the effects of antibiotics that once killed them, these antibiotic-resistant bacteria are often the root cause of the infections that trigger sepsis), weakened immune systems (more Americans are living with weakened immune systems, caused by HIV, cancer treatments or transplant drugs).

Non-poliovirus enteroviruses (EVs), consisting of coxsackievirues A and B, echoviruses, and newer, numbered EVs, are an important cause of numerous diseases including sepsis, neonatal illness and neonatal sepsis, with an incidence approximating or exceeding that of illness caused by *Streptococcus agalactiae,* herpes simplex virus (HSV), and symptomatic cytomegalovirus infections. Enteroviruses often associated with epidemic pleurodynia, hand-foot-and-mouth disease, herpangina, and poliomyelitis are caused almost exclusively by enteroviruses. Other disorders (eg, aseptic meningitis, myopericarditis) may be caused by enteroviruses or other organisms.

A subset of affected newborns develops severe disease manifesting with hepatitis, coagulopathy, myocarditis, pneumonitis, and/or meningoencephalitis. Infants with disease onset in the first few days of life, those lacking serotype-specific transplacentally-acquired neutralizing antibody, and those who are premature are at increased risk of severe illness. Hepatitis with coagulopathy and myocarditis are associated with mortality as high as 24-83% and 30-50%, respectively. Long-term morbidity including persistent hepatic and cardiac dysfunction and neurodevelopmental deficits may occur in survivors [1-5].

Until now antiviral therapy was not available for neonatal EV infections. The mainstay of treatment has been supportive care, which for severe infections includes intensive cardiorespiratory and blood product support. Small studies suggest potential benefit of intravenous immune globulin (IVIG), but efficacy remains unproven [6].

Pleconaril (3-{3,5-dimethyl-4-[3-(3-methylisoxazol-5-yl)propoxy]phenyl}-5-(trifluoromethyl) - 1,2,4-oxadiazole) comprised the first of a new class of antiviral agents designed to treat infections caused by picornaviruses. Pleconaril was rationally designed based on atomic resolution structures of drug/virus complexes, analyses of structure-activity relationships, screens for metabolic stability in liver microsomes, and extensive nonclinical safety testing. Pleconaril exhibited a broad activity against human respiratory picornavirus as shown during clinical studies of an oral dosage form (ViroPharma). However, it was found that oral delivery of this medication at 200-400 mg dose level introduces systematic exposure and causes side effects including the induction of CYP 3A, which resulted in negative complications including not limited to, an increase in steady-state plasma concentration of theophylline, and increase incidence of menstrual irregularities in women using oral contraceptives.

Until now, delivery of pleconaril has also proved problematic. In its original format, delivery of pleconaril using a nasal thixotropic formulation (ViroPharma IND Report, 2004), demonstrated that a 12 mg per day (nasal spray) may produce roughly similar clinical/antiviral effect to 400 mg oral dose without any significant systemic exposure. Although a thixotropic formulation becomes fluid upon agitation, when shear is not applied to the system, the formulation viscosity increases, which is not conducive to routine pharmaceutical use. Unfortunately there are several challenges that limit successful development of pleconaril nasal delivery. Most importantly, pleconaril is practically insoluble in water (solubility in water < 20 ng/ml at 25°C) and therefore has both low equilibrium concentration and slow dissolution rate in relevant bio-spaces where virus resides. The residence time of medication in the frontal turbinates and adenoids is relatively short for nasal sprays due to leakage and mucocilliary clearance whereas spraying into the blocked nostril or nose with rhinorrhea may further reduce absorption and sneezing may also expel the medication. As a result of the above limitations, despite its therapeutic potential, effective delivery of pleconaril has remained elusive until now.

The inventors herein have overcome the problems (undesirable systemic exposure, negative side effects, poor delivery etc) associated with the original formulation of pleconaril by developing and implementing a drug design strategy that optimizes particle design, formulation and delivery. In one aspect, the inventors have enabled site specific delivery of pleconaril, controlled by size of particles delivered (potentially bi or tri-modal distribution of pleconaril particle from sub-micron range approximately 1-50, 5-40, 10-35 and 30+ micron) or use of a novel delivery device to deliver to target areas where the locations of infection have the highest likelihood in resulting sepsis. The present invention discloses drug delivery advantages for enhanced therapeutic activity including a significantly reduced size of pleconaril particles wherein 90% of the particles are less than 1-100, 1-50, 1-10 or 1 microns constituting nanosuspensions with enhanced dissolution properties, increased therapeutic activity and reduced drug dose, but formulated in a manner that prevents these particles from indiscriminate delivery. More particularly, it has been discovered herein that administration of such suspensions allows for lower dosage levels than would be necessary to achieve a similar therapeutic response by other methods of delivery (e.g. micro-particulate delivery) for reduction of systemic side effects.

Disclosed herein are methods for the treatment of sepsis in a subject in need thereof, the method comprising, administering to the subject a composition comprising pleconaril. In an aspect, the sepsis is caused by enterovirus, in other aspect, the sepsis is neonatal sepsis. The sepsis in the subject may be related to the subject having an infection, asceptic meningitis, bone marrow transplantation, enterovirus myocarditis, chronic enterovirus meningoencephalitis, agammaglobulinemia, or hypogammaglobulinemia (CEMA). The symptoms associated may included, but are not limited to high body temperature (for example, over 99, 100, 101F or below 97, 96.8, 96 F, increased heart rate (for example, higher than 80-95 beats per minute), decreased urine output, abrupt change in mental status, decrease in platelet count, difficulty breathing, abnormal heart pumping function, abdominal pain, low blood pressure. Measurements such are these are known to those skilled in the art. In some aspects, symptoms of sepsis comprise congestive heart failure, chronic myocarditis, or dilated myocardiomyopathy.

Disclosed herein are methods for the treatment of sepsis in a subject in need thereof, the method comprising, administering to the subject a composition comprising pleconaril wherein the pleconaril is administered via oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, injectable administration, intravenous administration, intra-arterial administration, intramuscular administration, or intracardiac subcutaneous administration.

In an aspect, the composition comprising pleconaril comprises a parenteral formulation of pleconaril comprising pharmaceutically acceptable oils, pharmaceutically acceptable ester mixtures, saturated fatty acids, and/or combinations thereof.

In an aspect, the composition comprising pleconaril comprises a parenteral formulation of pleconaril comprising nanoemulsion of pleconaril and wherein the particle size of pleconaril comprises 50-500nm, 50-400nm, 50-300nm, 100-200nm, or 100nm. In additional aspects, the composition further comprises a pharmaceutically acceptable surfactant.

Disclosed herein are methods for the treatment of sepsis in a subject in need thereof, the methods comprising, administering to the subject a composition comprising pleconaril wherein the pleconaril is incorporated into polymeric phospholipid micelles. In some aspects, the polymeric phospholipid micelles comprise PEG, PEG conjugated with hydrophobic diacyl phospolipid, phosphatidylcholine, or egg phosphatidylcholine. The particle size of the polymeric phospholipid micelle comprises 5-100nm, 5-80nm, 10-50nm, or 10nm.

Disclosed herein are methods for the treatment of sepsis in a subject in need thereof, the methods comprising, administering to the subject a composition comprising pleconaril wherein the pleconaril comprises an oral formulation wherein the oral formulation comprises colloidal particles of pleconaril, optionally combined with carrier particles. In some aspects, the the carrier particles comprise silica particles, PEG, or hydroxypropylmethylcellulose. In certain aspects, the oral formulation of pleconaril described herein may be used to treat a subject for an extended period of time containing an antiviral agent or combination of antiviral agents and pharmaceutically acceptable salts thereof, where said formulation is comprises a solid dispersion which upon exposure to an aqueous environment releases the antiviral agent or agents as colloidal particles resulting in improved dissolution rate and bioavailability. In certain aspects, the dispersions can be made by heating drug and carrier to molten state and resolidification by cooling or dissolving components in appropriate solvent system and followed by evaporating solvent.

Disclosed herein are methods for the treatment of sepsis in a subject in need thereof, the methods comprising, administering to the subject a composition comprising pleconaril wherein the pleconaril comprises an oral formulation wherein the oral formulation comprises particles of pleconaril, incorporated into cyclic oligosaccharides. The cyclic oligosaccharides may comprise 2-hydroxypropyl-β-cyclodextrin, methyl-β-cyclodextrin, sulfobutyl-β-cyclodextrin. In certain aspects, the oral formulation described herein to treat subjects for an extended period of time comprises pleconaril or combination of antiviral agents and pharmaceutically acceptable salts thereof, where said formulation utilizes cyclic oligosaccharides to incorporate the antiviral agent(s) inside their hydrophobic cavity as a molecular encapsulation.

Disclosed herein are methods for the treatment of sepsis in a subject in need thereof, the methods comprising, administering to the subject a composition comprising pleconaril wherein the pleconaril comprises an oral formulation wherein the oral formulation comprises an oil-in-water microemulsion. In certain aspects, the oil in the oil-in-water microemulsion comprises a medium chain fatty acid triglyceride, a hydrogenated pharmaceutically acceptable oil, ethyl alcohol. In some aspects, the microemulsion is a self-microemulsifying formulation. In some aspects, the microemulsion particle size is 1-100nm, 5-90nm, 5-50nm or 8-12nm.

Disclosed herein are methods for the treatment of sepsis in a subject in need thereof, the methods comprising, administering to the subject a composition comprising pleconaril wherein the pleconaril comprises an oral formulation wherein the oral formulation comprises spray-dried emulsions of pleconaril combined with digestible oil. In an aspect, the digestible oil comprises soybean oil, sesame seed oil, cottonseed oil, safflower oil, oil comprising tocopherol, and/or oil comprising tocotrienol. In another aspect, the composition further comprises lecithin.

Disclosed herein are methods for the treatment of sepsis in a subject in need thereof, the methods comprising, administering to the subject a composition comprising pleconaril wherein the pleconaril is incorporated into an extended release composition. In an aspect, the composition is incorporated into a biodegradable gel. In another aspect,the biodegradable gel is a biodegradable thermal gel. In an aspect, the biodegradable gel comprises lactic acid, glycolic acid, PEG 1000 or 1450. In an aspect, treatment is extended by incorporating pleconaril and pharmaceutically acceptable salts thereof, into biodegradable thermal gels, wherein said gels utilize materials known to those skilled in the art such as lactic acid, glycolic acid, PEG 1000 or 1450, etc. The thermal gel once injected becomes a depot providing therapy for from 1-6 weeks and increases solubility from 200 -1000 fold.

Disclosed herein are methods for the treatment of sepsis in a subject in need thereof, the methods comprising, administering to the subject a composition comprising pleconaril wherein the compositions further comprise a pharmaceutically acceptable carrier. In an aspect, the composition comprising pleconaril comprises 1-600mg, 1-500mg, 1-300mg, 5-200mg, 10-100mg, 10-40mg pleconaril.

Disclosed herein are methods for the treatment of sepsis in a subject in need thereof, the methods comprising, administering to the subject a composition comprising pleconaril wherein the subject is considered at-risk. In an aspect, the at-risk subject comprises an infant, an individual aged 65 or older, an individual in an immunocomprised state, an individual undergoing a transplant procedure. In an aspect, the at-risk subject has rhinoviral pneumonia.

Disclosed herein is a composition comprising an improved formulation of pleconaril. In aspect, the composition comprises a parenteral formulation of pleconaril comprising pharmaceutically acceptable oils, pharmaceutically acceptable ester mixtures, saturated fatty acids, and combinations thereof. In an aspect, the formulation comprises nanoemulsions of pleconaril and wherein the particle size of pleconaril comprises 50-500nm, 50-400nm, 50-300nm, 100-200nm, or 100nm. In another aspect, the composition further comprises a pharmaceutically acceptable surfactant.

Disclosed herein is a composition comprising an improved formulation of pleconaril, wherein the composition comprising pleconaril is incorporated into polymeric phospholipid micelles. In an aspect, the polymeric phospholipid micelles comprise PEG, PEG conjugated with hydrophobic diacyl phospolipid, phosphatidylcholine, or egg phosphatidylcholine. In another aspect, the particle size of the polymeric phospholipid micelle comprises 5-100nm, 5-80nm, 10-50nm, or 10nm.

Disclosed herein is a composition comprising an improved formulation of pleconaril, wherein the composition comprising pleconaril comprises an oral formulation wherein the oral formulation comprises colloidal particles of pleconaril, optionally combined with carrier particles. In an aspect, the carrier particles comprise silica particles, PEG, or hydroxypropylmethylcellulose.

Disclosed herein is a composition comprising an improved formulation of pleconaril, wherein the composition comprising pleconaril comprises particles of pleconaril, incorporated into cyclic oligosaccharides. In an aspect, the cyclic oligosaccharides comprise 2-hydroxypropyl-β-cyclodextrin, methyl-β-cyclodextrin, sulfobutyl-β-cyclodextrin.

Disclosed herein is a composition comprising an improved formulation of pleconaril, wherein the composition comprising pleconaril comprises an oil-in-water microemulsion. In an aspect, the oil in the oil-in-water microemulsion comprises a medium chain fatty acid triglyceride, a hydrogenated pharmaceutically acceptable oil, ethyl alcohol. In another aspect, the the microemulsion is a self-microemulsifying formulation. In an aspect, the microemulsion particle size is 1-100nm, 5-90nm, 5-50nm or 8-12nm.

Disclosed herein is a composition comprising an improved formulation of pleconaril, wherein the composition comprising pleconaril comprises comprises an oral formulation wherein the oral formulation comprises spray-dried emulsions of pleconaril combined with digestible oil. In an aspect, the digestible oil comprises soybean oil, sesame seed oil, cottonseed oil, safflower oil, oil comprising tocopherol, and/or oil comprising tocotrienol. In an additional aspect, the composition comprises lecithin.

Disclosed herein is a composition comprising an improved formulation of pleconaril, wherein the composition comprising pleconaril is incorporated into an extended release composition. In an aspect, the composition comprising pleconaril is incorporated into a biodegradable gel. In an aspect, the biodegradable gel comprises lactic acid, glycolic acid, PEG 1000 or 1450.

Disclosed herein are compositions comprising an improved formulation of pleconaril, wherein the compositions comprising pleconaril further comprise a pharmaceutically acceptable carrier. In some aspects, the compositions comprising pleconaril comprises 1-600mg, 1-500mg, 1-300mg, 5-200mg, 10-100mg, 10-40mg per dosage, and are administered according to methods and protocols known to those skilled in the art, including once, twice or three times a day for a predetermined amount of time or until the condition of sepsis is alleviated. The compositions disclosed herein further comprise pharmaceutical salts of pleconaril. In addition, the compositions disclosed herein reduce CYP induction of antiviral agents.

Disclosed herein are compositions comprising an improved formulation of pleconaril, wherein the compositions comprising pleconaril further comprise one or more additional antiviral agents including, but not limited to, Abacavir, Aciclovir, Acyclovir, Adefovir, Amantadine, Aprenavir, Ampligen, Arbidol, Atazanavir, Atripla, Balavir, Boceprevirertet, cidofovir, Combivir, Dolutegravir, Darunavir, Dleavirdine, Didanosine, Docosanol, Edozudine, Efavirenz, Emtricitabine, Efuvirtide, Entecavir, Ecoliver, Famciclovir, Fomivirsen, Fosamprenavir, Foscarnet, Fosfonet, Fusion Inhibitors, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir Nucleoside analogues, Oseltamivir (Tamiflu), Peginterferon alfa-2a, Penciclovir, Peramivir, Podophyllotoxin, Protease inhibitors, Raltegravir, Reverse transcriptase inhibitor, Ribavirin, Rimantadine,Ritonavir, Pyramidine, Saquinavir, Sofosbuvir, Stavudine, Synergistic enhancer (antiretroviral), Telaprevir, Tenofovir, Tenofovir disoproxil, Tipranavir, Trifluridine, Trizivir, Tromantadine, Truvada, traporved, Valaciclovir (Valtrex), Valganciclovir, Vicriviroc, Vidarabine, Viramidine, Zalcitabine, Zanamivir (Relenza), or Zidovudine.

Also disclosed herein are uses of a disclosed compositions as investigational and/or research tools in the development and standardization of *in vitro* and *in vivo* test systems for evaluation in laboratory animals such as cats, dogs, rabbits, monkeys, rats and mice, as part of the search for new therapeutic approaches for the treatment of sepsis.

### Definitions

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

As used in the specification and the appended claims, the singular forms "a," "an" and "the" include plural referents unless the context clearly dictates otherwise.

The word "or" as used herein means any one member of a particular list and also includes any combination of members of that list.

Ranges can be expressed herein as from "about" one particular value, and/or to "about" another particular value. When such a range is expressed, a further aspect includes from the one particular value and/or to the other particular value. Similarly, when values are expressed as approximations, by use of the antecedent "about," it will be understood that the particular value forms a further aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

References in the specification and concluding claims to parts by weight of a particular element or component in a composition denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent (wt. %) of a component, unless specifically stated to the contrary, is based on the total weight of the formulation or composition in which the component is included.

As used herein, the terms "optional" or "optionally" means that the subsequently described event or circumstance can or can not occur, and that the description includes instances where said event or circumstance occurs and instances where it does not.

As used herein, the term "subject" refers to the target of administration, e.g., an animal. Thus, the subject of the herein disclosed methods can be a vertebrate, such as a mammal, a fish, a bird, a reptile, or an amphibian. Alternatively, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. In one aspect, the subject is a mammal. A patient refers to a subject afflicted with a disease or disorder. The term "patient" includes human and veterinary subjects. In some aspects of the disclosed methods, the subject has been diagnosed with a need for treatment for cardiac dysfunction, such as, for example, a cardiac ischemia/reperfusion event prior to the administering step.

As used herein, the term "treatment" refers to the medical management of a subject or a patient with the intent to cure, ameliorate, stabilize, or prevent a disease, pathological condition, or disorder, such as, for example, an injured myocardium. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease. In an aspect, the disease, pathological condition, or disorder is cardiac dysfunction, such as, for example, a cardiac ischemia/reperfusion event.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

As used herein, the term "diagnosed" means having been subjected to a physical examination by a person of skill, for example, a physician, and found to have a condition that can be diagnosed or treated by compositions or methods disclosed herein. For example, "diagnosed with sepsis" means having been subjected to a physical examination by a person of skill, for example, a physician, and found to have a condition that can be diagnosed or treated by a compound or composition that alleviates or ameliorates sepsis. Such a diagnosis can be in reference to a disorder, such as sepsis, and the like, as discussed herein.

As used herein, the phrase "identified to be in need of treatment for a disorder," or the like, refers to selection of a subject based upon need for treatment of the disorder. For example, a subject can be identified as having a need for treatment of a disorder (e.g., a disorder related to sepsis or neonatal sepsis) based upon an earlier diagnosis by a person of skill and thereafter subjected to treatment for the disorder. It is contemplated that the identification can, in one aspect, be performed by a person different from the person making the diagnosis. It is also contemplated, in a further aspect, that the administration can be performed by one who subsequently performed the administration.

As used herein, the terms "administering" and "administration" refer to any method of providing a pharmaceutical preparation to a subject. Such methods are well known to those skilled in the art and include, but are not limited to, intracardiac administration, oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

The term "contacting" as used herein refers to bringing a disclosed compound and a cell, target receptor, or other biological entity together in such a manner that the compound can affect the activity of the target (e.g., receptor, transcription factor, cell, etc.), either directly; i.e., by interacting with the target itself, or indirectly; i.e., by interacting with another molecule, co-factor, factor, or protein on which the activity of the target is dependent.

As used herein, the term "determining" can refer to measuring or ascertaining a quantity or an amount or a change in expression and/or activity level, e.g., of a nucleotide or transcript or polypeptide associated with enterovirus. For example, determining the amount of a disclosed enteroviral transcript or polypeptide in a sample as used herein can refer to the steps that the skilled person would take to measure or ascertain some quantifiable value of the transcript or polypeptide in the sample. The art is familiar with the ways to measure an amount of the disclosed nucleotides, transcripts, polypeptides, etc.

As used herein, the term "level" refers to the amount of a target molecule in a sample, e.g., a sample from a subject. The amount of the molecule can be determined by any method known in the art and will depend in part on the nature of the molecule (i.e., gene, mRNA, cDNA, protein, enzyme, etc.). The art is familiar with quantification methods for nucleotides (e.g., genes, cDNA, mRNA, etc) as well as proteins, polypeptides, enzymes, etc. It is understood that the amount or level of a molecule in a sample need not be determined in absolute terms, but can be determined in relative terms (e.g., when compare to a control or a sham or an untreated sample).

As used herein, the terms "effective amount" and "amount effective" refer to an amount that is sufficient to achieve the desired result or to have an effect on an undesired condition. For example, a "therapeutically effective amount" refers to an amount that is sufficient to achieve the desired therapeutic result or to have an effect on undesired symptoms, but is generally insufficient to cause adverse side affects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts.

By "modulate" is meant to alter, by increase or decrease. As used herein, a "modulator" can mean a composition that can either increase or decrease the expression level or activity level of a gene or gene product such as a peptide. Modulation in expression or activity does not have to be complete. For example, expression or activity can be modulated by about 10%, 20%, 30%, 40%, 50%, 60%, 70%, 80%, 90%, 95%, 99%, 100% or any percentage in between as compared to a control cell wherein the expression or activity of a gene or gene product has not been modulated by a composition.

As used herein "antiviral agents" is intended to include, but not limited to, antiviral agents known to those skilled in the art, including Abacavir, Aciclovir, Acyclovir, Adefovir, Amantadine, Aprenavir, Ampligen, Arbidol, Atazanavir, Atripla, Balavir, Boceprevirertet, cidofovir, Combivir, Dolutegravir, Darunavir, Dleavirdine, Didanosine, Docosanol, Edozudine, Efavirenz, Emtricitabine, Efuvirtide, Entecavir, Ecoliver, Famciclovir, Fomivirsen, Fosamprenavir, Foscarnet, Fosfonet, Fusion Inhibitors, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir Nucleoside analogues, Oseltamivir (Tamiflu), Peginterferon alfa-2a, Penciclovir, Peramivir, Podophyllotoxin, Protease inhibitor (pharmacology), Raltegravir, Reverse transcriptase inhibitor, Ribavirin, Rimantadine,Ritonavir, Pyramidine, Saquinavir, Sofosbuvir, Stavudine, Synergistic enhancer (antiretroviral), Tea tree oil, Telaprevir, Tenofovir, Tenofovir disoproxil, Tipranavir, Trifluridine, Trizivir, Tromantadine, Truvada, traporved, Valaciclovir (Valtrex), Valganciclovir, Vicriviroc, Vidarabine, Viramidine, Zalcitabine, Zanamivir (Relenza), and Zidovudine.

As used herein, "EC₅₀," is intended to refer to the concentration or dose of a substance (e.g., a compound or a drug) that is required for 50% enhancement or activation of a biological process, or component of a process, including a protein, subunit, organelle, ribonucleoprotein, etc. EC₅₀ also refers to the concentration or dose of a substance that is required for 50% enhancement or activation *in vivo,* as further defined elsewhere herein. Alternatively, EC₅₀ can refer to the concentration or dose of compound that provokes a response halfway between the baseline and maximum response. The response can be measured in a *in vitro* or *in vivo* system as is convenient and appropriate for the biological response of interest. For example, the response can be measured *in vitro* using cultured endothelial cells or in an *ex vivo* organ culture system with isolated cells. Alternatively, the response can be measured *in vivo* using an appropriate research model such as rodent, including mice and rats. The mouse or rat can be an inbred strain with phenotypic characteristics of interest such as, for example, susceptibility to sepsis. As appropriate, the response can be measured in a transgenic or knockout mouse or rat wherein a gene or genes has been introduced or knocked-out, as appropriate, to replicate a disease process.

As used herein, "IC₅₀," is intended to refer to the concentration or dose of a substance (e.g., a compound or a drug) that is required for 50% inhibition or diminution of a biological process, or component of a process, including a protein, subunit, organelle, ribonucleoprotein, etc. IC₅₀ also refers to the concentration or dose of a substance that is required for 50% inhibition or diminution *in vivo,* as further defined elsewhere herein. Alternatively, IC₅₀ also refers to the half maximal (50%) inhibitory concentration (IC) or inhibitory dose of a substance. The response can be measured in a *in vitro* or *in vivo* system as is convenient and appropriate for the biological response of interest. For example, the response can be measured *in vitro* for disorders such as myopericarditis using cultured cardiac cells or in an *ex vivo* organ culture system with isolated cardiac cells (e.g., cardiomyocytes, vascular smooth muscle cells, endothelial cells, etc). Alternatively, the response can be measured *in vivo* using an appropriate research model such as rodent, including mice and rats. The mouse or rat can be an inbred strain with phenotypic characteristics of interest such as, for example, susceptibility to sepsis. As appropriate, the response can be measured in a transgenic or knockout mouse or rat wherein the a gene or genes has been introduced or knocked-out, as appropriate, to replicate a disease process.

The term "pharmaceutically acceptable" describes a material that is not biologically or otherwise undesirable, i.e., without causing an unacceptable level of undesirable biological effects or interacting in a deleterious manner. As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose. Desirably, at least 95% by weight of the particles of the active ingredient have an effective particle size in the range of 0.01 to 10 micrometers.

Disclosed are the components to be used to prepare a composition of the invention as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds can not be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the invention. Thus, if there are a variety of additional steps that can be performed it is understood that each of these additional steps can be performed with any specific embodiment or combination of embodiments of the methods of the invention.

The present invention can be understood more readily by reference to the following detailed description of the invention and the Examples included therein.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

### EXAMPLES

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. However, those of skill in the art should, in light of the present disclosure, appreciate that many changes can be made in the specific embodiments which are disclosed and still obtain a like or similar result without departing from the spirit and scope of the invention.

Efforts have been made to ensure accuracy with respect to numbers (*e.g*., amounts, temperature, etc.), but some errors and deviations should be accounted for. Unless indicated otherwise, parts are parts by weight, temperature is in °C or is at ambient temperature, and pressure is at or near atmospheric.

### Example 1

### Design and implementation of study to investigate efficacy of Pleconaril as antiviral therapy for neonatal enterovirus infections

### 1. Study population

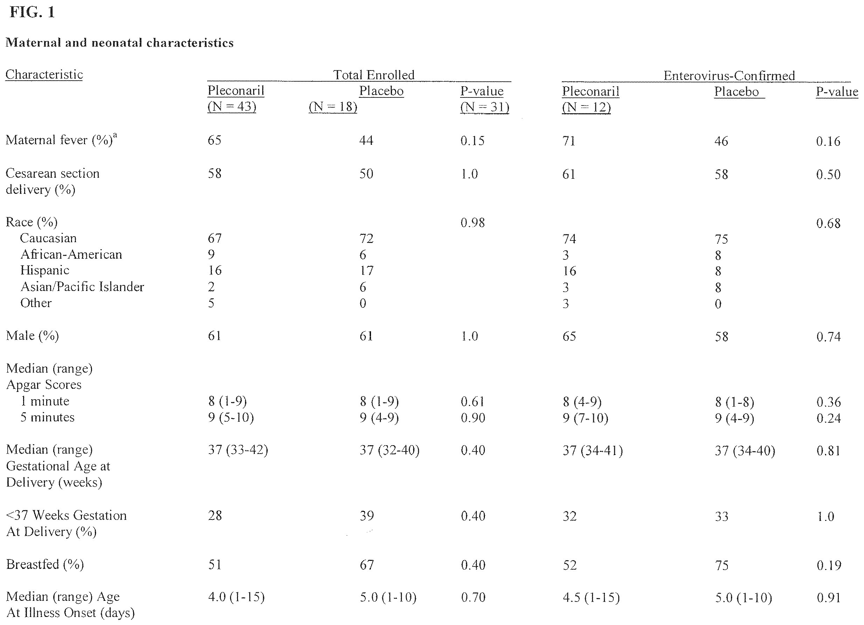

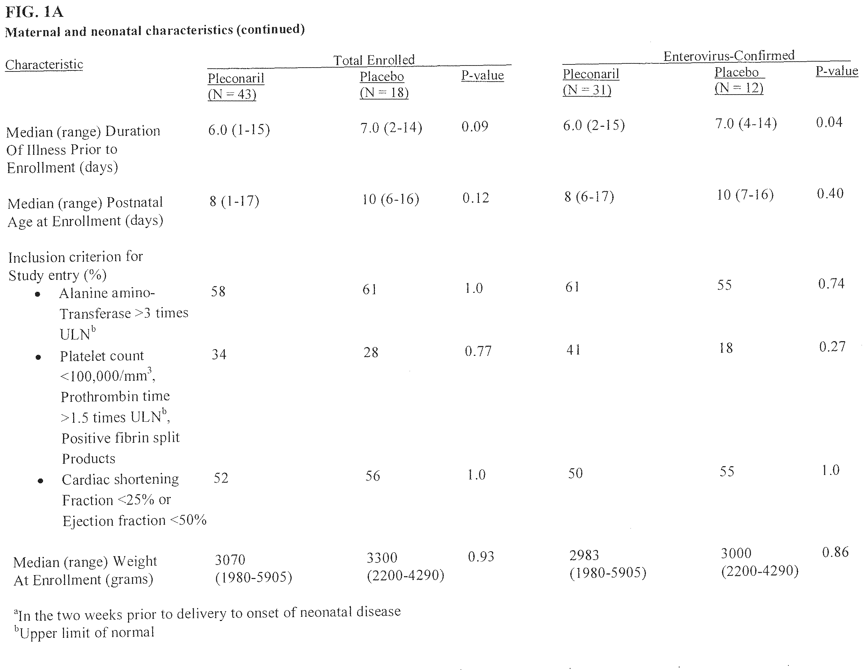

Sixty-one subjects were enrolled; 43 randomized to the treatment group and 18 to the placebo group (Figure 1). Of these, 43 (70%) were confirmed EV-positive: 31 in the treatment group and 12 in the placebo group. The study was terminated prior to full accrual due to expiration of study drug. The frequency of premature treatment and protocol discontinuation did not differ between study groups (9% v. 28%, p = 0.11, and 56% v. 72%, p = 0.27, respectively); however, there was a difference in reasons for protocol discontinuation (p = 0.04): death in 10/43 (23%) subjects in the treatment group v. 8/18 (44%) in the placebo group and lost-to-follow-up in 9/43 (21%) subjects in the treatment group v. 1/18 (6%) in the placebo group. Neonates with suspected EV sepsis (hepatitis, coagulopathy, and/or myocarditis) were enrolled at 19 centers from June 1999-December 2010.

### 2. Study Design

A 2:1 study drug: placebo randomization with randomly permuted block sizes was used. Randomization codes were accessible only to biostatistical personnel at the coordinating center. Subjects initially received 5.0 mg/kg/dose of a 40 mg/mL liquid formulation of pleconaril (ViroPharma, Inc.; Exton, PA) or matching placebo identical in composition except lacking active study drug. The liquid formulation expired on May 31, 2002, after which the formulation was changed by the manufacturer to a 40 mg/mL suspension. Subjects received 8.5 mg/kg/dose of pleconaril suspension (shown to provide similar drug exposure as 5.0 mg/kg/dose of pleconaril liquid) or matching placebo. Both pleconaril formulations and matching placebos were administered every 8 hours x 7 days in 1.0 mL of breast milk or formula by mouth or orogastric tube and were given to subjects who were otherwise NPO. Subjects were permitted to receive antibiotics, acyclovir, cardiotropic medications, and IVIG. Subjects for whom an etiology for illness other than EV infection was documented after enrollment had study drug/placebo discontinued.

### 3. Sample Size and Statistical Analysis

A target sample size of 45 subjects with confirmed EV disease, with an expectation that approximately 67% of enrolled subjects would be confirmed to be EV-infected, was chosen to provide sufficient power to detect a reduction in viral shedding from the oropharynx from 96% to 70% on day 5. EV-confirmed subjects (culture- or PCR-positive) were included in virologic and clinical efficacy analyses, all enrolled subjects were included in safety analyses, and pleconaril recipients for whom pharmacokinetic data were obtained (whether or not EV-infected) were included in pharmacokinetic analyses. Survival analyses were conducted on the total enrolled cohort and on EV-confirmed subjects.

Fisher's Exact Test was utilized for comparisons of categorical factors and the Kruskal-Wallis and Mann Whitney Tests for continuous factors, with medians and ranges presented. Kaplan-Meier curves and log-rank statistics were utilized to assess differences in duration of culture and PCR positivity and survival between study groups. Changes in toxicity grades from baseline and adverse event rates were compared by chi-square tests.

### Example 2: Virological efficacy of Pleconaril

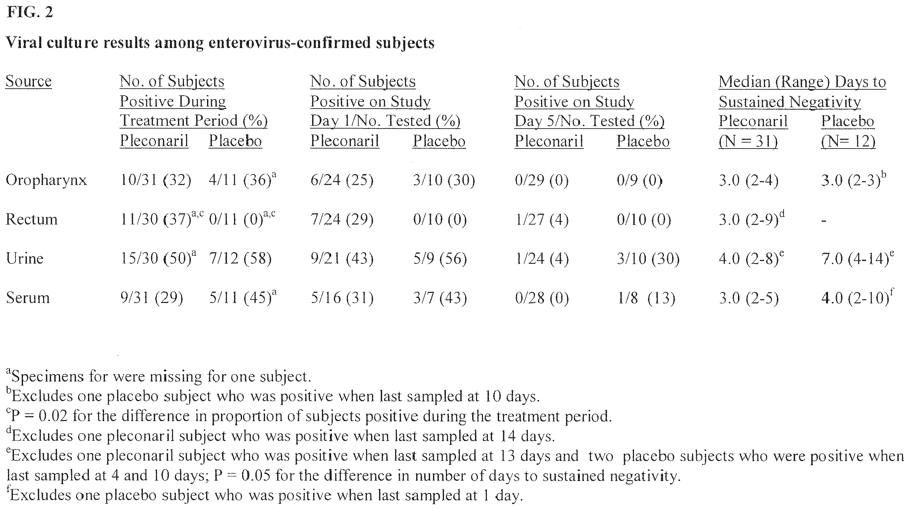

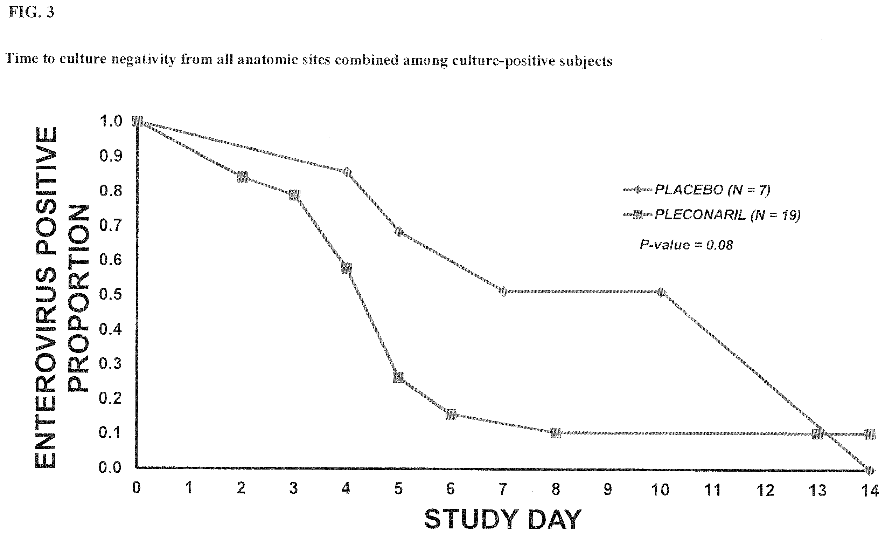

Nineteen subjects in the treatment group and seven in the placebo group were culture-positive from any site during the treatment period, and 31 subjects in the treatment group and 12 in the placebo group were PCR-positive from any site during the treatment period. All culture-positive subjects were also PCR-positive. Six of 24 (25%) EV-confirmed subjects in the treatment group and 3/10 (30%) in the placebo group were culture-positive from the oropharynx on day 1, and no subjects shed cultivable virus from the oropharynx on day 5 (Figure 2). The median time to sustained oropharyngeal culture negativity was 3.0 days in both the treatment and placebo groups. Among subjects who were culture-positive from any site, those in the treatment group became culture-negative from all sites faster than those in the placebo group, with the difference approaching significance (p = 0.08); median time to culture-negativity among culture-positive subjects in the treatment group was 4.0 days v. 7.0 days in the placebo group (Figure 3). A similar pattern was observed for urine (median time to sustained culture-negativity = 4.0 v. 7.0 days, respectively; p=0.05; Figure 3), but not other individual sites.

### Example 3: Clinical efficacy of Pleconaril

Among all enrolled subjects, 10/43 (23%) in the treatment group and 8/18 (44%) in the placebo group died, and cumulative survival over two months was greater in the treatment group (p = 0.02). Among EV-confirmed subjects, 7/31 (23%) in the treatment group and 5/12 (42%) in the placebo group died, with trends in cumulative survival favoring the treatment group. Cumulative survival in the treatment group continued to exceed that in the placebo group over 18 months, p = 0.07 among all subjects and p = 0.23 among EV-confirmed subjects.

### 1. Materials and Methods

Specimens for EV culture and polymerase chain reaction (PCR) were obtained from the oropharynx, rectum, urine, and serum on days 1-5, 7, 10, and 14. Plasma for pharmacokinetic analysis was obtained on day 1 (2, 4, and 8 hours after study drug administration) and days 3 and 7 (prior to and 2 and 4 hours after administration). Hematology and chemistry tests were obtained on study days 1, 3, 5, 7, 10, and 14 and urinalyses on days 1 and 7. Persisting abnormalities were followed at visits at 2, 6, 12, 18, and 24 months. Adverse events were recorded at each evaluation, and relationship to study treatment was determined by site investigators.

The primary endpoint was the percentage of patients with a positive viral culture from the oropharynx five days after beginning study drug. Secondary endpoints included duration of culture positivity from oropharynx, rectum, urine and serum; changes in laboratory values reflecting resolution or progression of EV disease or drug toxicity; duration of hospitalization; time to resolution of organ abnormalities; survival; and pleconaril pharmacokinetics. Tertiary endpoints included duration of blood product (fresh frozen plasma, packed red blood cells, platelets, other products) and inotropic support.

### Example 4: Safety analysis of Pleconaril

Eighty-six percent of enrolled subjects in the treatment group and 94% in the placebo group experienced adverse events, of whom only 3% and 18%, respectively, had events judged possibly/probably treatment-related. The number of events/subject and severity distribution were similar between groups. Only one subject (placebo group) had a serious adverse event considered possibly/probably treatment-related. No subjects had treatment withdrawn due to adverse events other than identification of herpes simplex virus as the cause of illness (3) or death (5).

As demonstrated in the Examples above, viral cultures had unexpectedly low yield. Indicators of potential virologic efficacy were positive: time to culture negativity of all sites combined was shorter in pleconaril group and persistence of PCR positivity from OP shorter in pleconaril group. In addition, differences in survival suggest potential clinical efficacy. Similar AE profiles between groups and few AEs attributed to study treatment demonstrate safety. Pleconaril concentrations exceeded IC₉₀ *after* D1: concentrations on Dl <IC₉₀ in 41% suggest need for loading dose. Signals of efficacy and absence of safety concerns demonstrate the efficacy of pleconaril for the treatment of neonatal EV disease.

### REFERENCES

1. Abzug MJ. Prognosis for neonates with enterovirus hepatitis and coagulopathy. Pediatr Infect Dis J 2001;20:758-63.
2. Abzug MJ. Presentation, diagnosis, and management of enterovirus infection in neonates. Paediatr Drugs 2004;6:1-10.
3. Abzug MJ. The enteroviruses: problems in need of treatments. J Infection 2014;68:S108-14.
4. Freund MW, Kleinveld G, Krediet TG, van Loon AM, Verboon-Maciolek MA. Prognosis for neonates with enterovirus myocarditis. Arch Dis Child Fetal Neonatal Ed 2010;95:F206-12.
5. Tebruegge M, Curtis N. Enterovirus infections in neonates. Semin Fetal Neonatal Med 2009;14:222-7.
6. Abzug MJ, Keyserling HL, Lee ML, Levin MJ, Rotbart HA. Neonatal enterovirus infection: virology, serology, and effects of intravenous immune globulin. Clin Infect Dis 1995;20:1201-6.
7. Abzug MJ, Cloud G, Bradley J, et al. Double blind placebo-controlled trial of pleconaril in infants with enterovirus meningitis. Pediatr Infect Dis J. 2003;22:335-41.
8. Desmond RA, Accortt NA, Talley L, Villano SA, Soong SJ, Whitley RJ. Enteroviral meningitis: natural history and outcome of pleconaril therapy. Antimicrob Agents Chemother 2006;50:2409-14.
9. Hayden FG, Herrington DT, Coats TL, et al. Efficacy and safety of oral pleconaril for treatment of colds due to picornaviruses in adults: results of 2 double-blind, randomized, placebo-controlled trials. Clin Infect Dis 2003;36:1523-32.

### STATEMENTS

### What is disclosed is:

1. A method for the treatment of sepsis in a subject in need thereof, the method comprising, administering to the subject a composition comprising pleconaril.
2. The method of statement 1, wherein sepsis is caused by enterovirus.
3. The method of statement 1, wherein sepsis is neonatal sepsis.
4. The method of statement 1, wherein sepsis in the subject is related to the subject having an infection, asceptic meningitis, bone marrow transplantation, enterovirus myocarditis, chronic enterovirus meningoencephalitis, agammaglobulinemia, or hypogammaglobulinemia (CEMA).
5. The method of statement 1, wherein symptoms of sepsis comprise high body temperature, increased heart rate, decreased urine output, abrupt change in mental status, decrease in platelet count, difficulty breathing, abnormal heart pumping function, abdominal pain, low blood pressure.
6. The method of statement 1, wherein symptoms of sepsis comprise congestive heart failure, chronic myocarditis, or dilated myocardiomyopathy
7. The method of statement 1, wherein the pleconaril is administered via oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, injectable administration, intravenous administration, intra-arterial administration, intramuscular administration, or intracardiac subcutaneous administration.
8. The method of statement 1, wherein the composition comprising pleconaril comprises a parenteral formulation of pleconaril comprising pharmaceutically acceptable oils, pharmaceutically acceptable ester mixtures, saturated fatty acids, hydrofluorocarbons, and/or combinations thereof.
9. The method of statement 8, wherein the composition comprising pleconaril comprises a parenteral formulation of pleconaril comprising nanoemulsion of pleconaril and wherein the particle size of pleconaril comprises 50-500nm, 50-400nm, 50-300nm, 100-200nm, or 100nm.
10. The method of statement 8 or 9, wherein the composition comprising pleconaril comprises a parenteral formulation of pleconaril comprising nanosuspension of pleconaril and wherein the particle size of pleconaril comprises 100nm.
11. The method of statement 10, further comprising a pharmaceutically acceptable surfactant.
12. The method of statement 1, wherein the composition comprising pleconaril is incorporated into polymeric phospholipid micelles.
13. The method of statement 12, wherein polymeric phospholipid micelles comprise PEG, PEG conjugated with hydrophobic diacyl phospolipid, phosphatidylcholine, or egg phosphatidylcholine .
14. The method of statement 13, wherein the particle size of the polymeric phospholipid micelle comprises 5-100nm, 5-80nm, 10-50nm, or 10nm.
15. The method of statement 1, wherein the composition comprising pleconaril comprises an oral formulation wherein the oral formulation comprises colloidal particles of pleconaril, optionally combined with carrier particles.
16. The method of statement 15, wherein the carrier particles comprise silica particles, PEG, or hydroxypropylmethylcellulose.
17. The method of statement 1, wherein the composition comprising pleconaril comprises an oral formulation wherein the oral formulation comprises particles of pleconaril, incorporated into cyclic oligosaccharides.
18. The method of statement 17, wherein the cyclic oligosaccharides comprise 2-hydroxypropyl-β-cyclodextrin, methyl-β-cyclodextrin, sulfobutyl-β-cyclodextrin.
19. The method of statement 1, wherein the composition comprising pleconaril comprises an oral formulation wherein the oral formulation comprises an oil-in-water microemulsion.
20. The method of statement 19, wherein the oil in the oil-in-water microemulsion comprises a medium chain fatty acid triglyceride, a hydrogenated pharmaceutically acceptable oil, ethyl alcohol.
21. The method of statement 20, wherein the microemulsion is a self-microemulsifying formulation.
22. The method of statement 21, wherein the microemulsion particle size is 1-100nm, 5-90nm, 5-50nm or 8-12nm.
23. The method of statement 22, wherein the composition comprising pleconaril comprises an oral formulation wherein the oral formulation comprises spray-dried emulsions of pleconaril combined with digestible oil.
24. The method of statement 23, wherein the digestible oil comprises soybean oil, sesame seed oil, cottonseed oil, safflower oil, oil comprising tocopherol, and/or oil comprising tocotrienol.
25. The method of statement 23 or 24, further comprising lecithin.
26. The method of statement 1, wherein the composition comprising pleconaril is incorporated into an extended release composition.
27. The method of statement 26 wherein the composition comprising pleconaril is incorporated into a biodegradable gel.
28. The method of statement 27, wherein the biodegradable gel is a biodegradable thermal gel.
29. The method of statement 28, wherein the biodegradable gel comprises lactic acid, glycolic acid, PEG 1000 or 1450.
30. The method of statements 1-29, wherein the composition comprising pleconaril further comprises a pharmaceutically acceptable carrier.
31. The method of statements 1-29, wherein the composition comprising pleconaril comprises 1-600mg, 1-500mg, 1-300mg, 5-200mg, 10-100mg, 10-40mg.
32. The method of statements 1-29, wherein the subject is considered at-risk.
33. The method of statement 32, wherein the at-risk subject comprises an infant, an individual aged 65 or older, and individual with an immunocomprised state, an individual undergoing a transplant procedure.
34. The method of statement 33, wherein the at-risk subject has rhinoviral pneumonia. Reduce CYP induction of antiviral agents and Incorporation and pharmaceutically salts thereof, through use of the parenteral formulations described above.
35. A composition comprising an improved formulation of pleconaril.
36. The composition of statement 35, wherein the composition comprises a parenteral formulation of pleconaril comprising pharmaceutically acceptable oils, pharmaceutically acceptable ester mixtures, saturated fatty acids, hydrofluorocarbons, and combinations thereof.
37. The composition of statement 36, wherein the formulation comprises nanoemulsions of pleconaril and wherein the particle size of pleconaril comprises 50-500nm, 50-400nm, 50-300nm, 100-200nm, or 100nm.
38. The composition of statement 37, further comprising a pharmaceutically acceptable surfactant.
39. The composition of statement 35, wherein the composition comprising pleconaril is incorporated into polymeric phospholipid micelles.
40. The composition of statement 39, wherein polymeric phospholipid micelles comprise PEG, PEG conjugated with hydrophobic diacyl phospolipid, phosphatidylcholine, or egg phosphatidylcholine.
41. The composition of statement 40, wherein the particle size of the polymeric phospholipid micelle comprises 5-100nm, 5-80nm, 10-50nm, or 10nm.
42. The composition of statement 35, wherein the composition comprising pleconaril comprises an oral formulation wherein the oral formulation comprises colloidal particles of pleconaril, optionally combined with carrier particles.
43. The composition of statement 42, wherein the carrier particles comprise silica particles, PEG, or hydroxypropylmethylcellulose.
44. The composition of statement 35, wherein the composition comprising pleconaril comprises an oral formulation wherein the oral formulation comprises particles of pleconaril, incorporated into cyclic oligosaccharides.
45. The composition of statement 41, wherein the cyclic oligosaccharides comprise 2-hydroxypropyl-β-cyclodextrin, methyl-β-cyclodextrin, sulfobutyl-β-cyclodextrin.
46. The composition of statement 35, wherein the composition comprising pleconaril comprises an oral formulation wherein the oral formulation comprises an oil-in-water microemulsion.
47. The composition of statement 46, wherein the oil in the oil-in-water microemulsion comprises a medium chain fatty acid triglyceride, a hydrogenated pharmaceutically acceptable oil, ethyl alcohol.
48. The composition of statement 47, wherein the microemulsion is a self-microemulsifying formulation.
49. The composition of statement 46, wherein the microemulsion particle size is 1-100nm, 5-90nm, 5-50nm or 8-12nm.
50. The composition of statement 35, wherein the composition comprising pleconaril comprises an oral formulation wherein the oral formulation comprises spray-dried emulsions of pleconaril combined with digestible oil.
51. The composition of statement 50, wherein the digestible oil comprises soybean oil, sesame seed oil, cottonseed oil, safflower oil, oil comprising tocopherol, and/or oil comprising tocotrienol.
52. The composition of statement 51, further comprising lecithin.
53. The composition of statement 35, wherein the composition comprising pleconaril is incorporated into an extended release composition.
54. The composition of statement 53, wherein the composition comprising pleconaril is incorporated into a biodegradable gel.
55. The composition of statement 54, wherein the biodegradable gel comprises lactic acid, glycolic acid, PEG 1000 or 1450.
56. The composition of statements 35-55, wherein the composition comprising pleconaril further comprises a pharmaceutically acceptable carrier.
57. The composition of statements 35-55, wherein the composition comprising pleconaril comprises 1-600mg, 1-500mg, 1-300mg, 5-200mg, 10-100mg, 10-40mg.
58. The composition of statements 35-55 further comprising pharmaceutical salts of pleconaril.
59. The composition of statements 35-58 wherein the formulation is designed for delivery via oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, injectable administration, intravenous administration, intra-arterial administration, intramuscular administration, or intracardiac subcutaneous administration.
60. The composition of statements 35-58 wherein the formulation further comprises one or more additional antiviral agents comprising Abacavir, Aciclovir, Acyclovir, Adefovir, Amantadine, Aprenavir, Ampligen, Arbidol, Atazanavir, Atripla, Balavir, Boceprevirertet, cidofovir, Combivir, Dolutegravir, Darunavir, Dleavirdine, Didanosine, Docosanol, Edozudine, Efavirenz, Emtricitabine, Efuvirtide, Entecavir, Ecoliver, Famciclovir, Fomivirsen, Fosamprenavir, Foscarnet, Fosfonet, Fusion Inhibitors, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir Nucleoside analogues, Oseltamivir (Tamiflu), Peginterferon alfa-2a, Penciclovir, Peramivir, Podophyllotoxin, Protease inhibitors, Raltegravir, Reverse transcriptase inhibitor, Ribavirin, Rimantadine,Ritonavir, Pyramidine, Saquinavir, Sofosbuvir, Stavudine, Synergistic enhancer (antiretroviral), Telaprevir, Tenofovir, Tenofovir disoproxil, Tipranavir, Trifluridine, Trizivir, Tromantadine, Truvada, traporved, Valaciclovir (Valtrex), Valganciclovir, Vicriviroc, Vidarabine, Viramidine, Zalcitabine, Zanamivir (Relenza), or Zidovudine.

## Claims

1. A composition consisting essentially of a pleconaril suspension admixed with breast milk or formula, to be administered orally or with an orogastric tube, for use in the treatment of neonatal enteroviral sepsis.

2. The composition of claim 1, wherein the pleconaril comprises particles and wherein 90% of the pleconaril particles are 1 - 50 microns.

3. The composition of claim 1, wherein the parenteral composition further comprises pharmaceutically acceptable oils, pharmaceutically acceptable ester mixtures, saturated fatty acids, hydrofluorocarbons, and / or combinations thereof.

4. The composition of claim 1, wherein the composition comprising pleconaril is incorporated into polymeric phospholipid micelles; wherein polymeric phospholipid micelles comprise PEG, PEG conjugated with hydrophobic diacyl phospolipid, phosphatidylcholine, or egg phosphatidylcholine; wherein the particle size of the polymeric phospholipid micelle comprises 5-100 nm, 5-80 nm, 10-50 nm, or 10 nm.

5. The composition of claim 1, wherein the pleconaril suspension comprises colloidal particles of pleconaril, optionally combined with carrier particles; wherein the carrier particles comprise silica particles, PEG, or hydroxypropylmethylcellulose.

6. The composition of claim 1, wherein the pleconaril suspension comprises particles of pleconaril, incorporated into cyclic oligosaccharides; wherein the cyclic oligosaccharides comprise 2-hydroxypropyl-.beta.-cyclodextrin, methyl-.beta.-cyclodextrin, or sulfobutyl-beta.-cyclodextrin.

7. The composition of claim 6, wherein the pleconaril suspension comprises an oil-in-water microemulsion; wherein the oil in the oil-in-water microemulsion comprises a medium chain fatty acid triglyceride, a hydrogenated pharmaceutically acceptable oil, ethyl alcohol; wherein the microemulsion particle size is 1-100 nm, 5-90 nm, 5-50 nm or 8-12 nm.

8. The composition of claim 7, wherein the pleconaril suspension comprises spray-dried emulsions of pleconaril combined with digestible oil.

9. The composition of claim 1, further comprising pharmaceutical salts of pleconaril.

10. The composition of claim 1, wherein the composition further comprises one or more additional antiviral agents comprising Abacavir, Aciclovir, Acyclovir, Adefovir, Amantadine, Aprenavir, Ampligen, Arbidol, Atazanavir, Atripla, Balavir, Boceprevirertet, cidofovir, Combivir, Dolutegravir, Darunavir, Dleavirdine, Didanosine, Docosanol, Edozudine, Efavirenz, Emtricitabine, Efuvirtide, Entecavir, Ecoliver, Famciclovir, Fomivirsen, Fosamprenavir, Foscarnet, Fosfonet, Fusion Inhibitors, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Nelfinavir, Nevirapine, Nexavir Nucleoside analogues, Oseltamivir (Tamiflu), Peginterferon alfa-2a, Penciclovir, Peramivir, Podophyllotoxin, Protease inhibitors, Raltegravir, Reverse transcriptase inhibitor, Ribavirin, Rimantadine, Ritonavir, Pyramidine, Saquinavir, Sofosbuvir, Stavudine, Synergistic enhancer (antiretroviral), Telaprevir, Tenofovir, Tenofovir disoproxil, Tipranavir, Trifluridine, Trizivir, Tromantadine, Truvada, traporved, Valaciclovir (Valtrex), Valganciclovir, Vicriviroc, Vidarabine, Viramidine, Zalcitabine, Zanamivir (Relenza), or Zidovudine.

11. The composition of claim 1, wherein the neonatal sepsis arises from aseptic meningitis, bone marrow transplantation, enterovirus myocarditis, chronic enterovirus meningoencephalitis, agammaglobulinemia, or hypogammaglobulinemia.

12. The composition of claim 1, wherein symptoms of neonatal sepsis comprise high body temperature, decreased urine output, abrupt change in mental status, decrease in platelet count, difficulty breathing, abdominal pain, multi-organ involvement, or hepatic dysfunction.
